# EUROPÄISCHE PATENTSCHRIFT

(11) **EP 3 359 077 B1**
(45) Veröffentlichungstag und Bekanntmachung des Hinweises auf die Patenterteilung: **06.12.2023**
(21) Anmeldenummer: 16781364.1
(22) Anmeldetag: 10.10.2016
(51) Int. Cl.: A61B 90/00, A61B 34/20

(54) **CHIRURGISCHES MARKERELEMENT, CHIRURGISCHE REFERENZIERUNGSEINHEIT UND CHIRURGISCHES NAVIGATIONSSYSTEM**
SURGICAL MARKER ELEMENT, SURGICAL REFERENCING UNIT, AND SURGICAL NAVIGATION SYSTEM
ÉLÉMENT MARQUEUR CHIRURGICAL, UNITÉ DE RÉFÉRENCEMENT CHIRURGICALE ET SYSTÈME DE NAVIGATION CHIRURGICAL

(30) Priorität: 09.10.2015 DE 102015117239
(43) Veröffentlichungstag der Anmeldung: 15.08.2018
(73) Patentinhaber: Aesculap AG, 78532 Tuttlingen (DE)
(72) Erfinder: BROERS, Holger, 26670 Uplengen-Spols (DE); GÖGGELMANN, Andreas, 78269 Volkertshausen (DE); PFEIFER, Tobias, 78713 Waldmössingen (DE)
(74) Vertreter: Hoeger, Stellrecht & Partner Patentanwälte mbB
(86) Internationale Anmeldenummer: PCT/EP2016/074186
(87) Internationale Veröffentlichungsnummer: WO 2017/060522

(56) Entgegenhaltungen:
- EP-A1- 1 639 958
- WO-A1-2013/126361
- DE-A1-102008 022 254
- DE-A1-102010 036 719
- US-A1- 2007 183 041

## Beschreibung

Die vorliegende Erfindung betrifft ein Markerelement, insbesondere ein medizinisches oder chirurgisches Markerelement, für eine Referenzierungseinheit eines Navigationssystems, welches Markerelement elektromagnetische Strahlung reflektierend ausgebildet ist, wobei das Markerelement eine Schicht umfassend eine Mehrzahl retroreflektierender Elemente und einen Träger für die Schicht retroreflektierender Elemente umfasst.

Ferner betrifft die vorliegende Erfindung eine Referenzierungseinheit, insbesondere eine medizinische oder chirurgische Referenzierungseinheit, deren Position und/oder Orientierung im Raum mit einem chirurgischen Navigationssystem detektierbar ist, mit mindestens einem chirurgischen Markerelement.

Außerdem betrifft die vorliegende Erfindung ein Navigationssystem, insbesondere ein medizinisches oder chirurgisches Navigationssystem, mit mindestens einer mindestens drei Markerelemente umfassenden Referenzierungseinheit und mit mindestens einer Nachweisvorrichtung zum Detektieren der Position und/oder der Orientierung der Referenzierungseinheit im Raum, wobei die Referenzierungseinheit mindestens ein chirurgisches Markerelement umfasst.

Markerelemente, Referenzierungseinheiten und Navigationssysteme in Form chirurgischer oder medizinischer Markerelemente, Referenzierungseinheiten und Navigationssysteme sind beispielsweise aus der DE 10 2007 011 595 A1 bekannt. Insbesondere kommen dabei als Markerelemente Kugeln zum Einsatz, die mit elektromagnetische Strahlung, insbesondere Infrarotstrahlung, reflektierenden Spezialfolien überzogen sind. Zur Bestimmung von einer Position und/oder Orientierung der Referenzierungseinheit im Raum wird mit den Navigationssystemen das Prinzip der Triangulation verwirklicht. Auf Basis von Winkelmessungen und eines Maßstabes, beispielsweise der Orientierung zweier Detektoren in Form von Kameras, werden dreidimensionale Informationen der Referenzierungseinheit berechnet. Merkmale der Referenzierungseinheit werden dabei in den Bilddaten identifiziert und zugeordnet. Bekannt ist es auch, abhängig von Anforderungen, künstliche Signalisierungen an der Referenzierungseinheit anzubringen und diese zu vermessen. Ein besonders gutes Verhältnis zwischen zu messenden Merkmalen der Referenzierungseinheit und einem beispielsweise in einem Operationssaal vorhandenen Hintergrund wird durch selbstleuchtende Markerelemente, auch als sogenannte aktive Markerelemente bezeichnet, erreicht.

Ein Problem bei den bekannten passiven Markerelementen, die mit speziellen Folien beschichtet sind, ist insbesondere, dass diese nur diffus reflektieren, sodass stets nur ein kleiner Teil der vom Navigationssystem ausgesandten elektromagnetischen Strahlung zu diesem zurück gesendet wird. Dies liegt insbesondere daran, dass bei einer spiegelnden Oberfläche nur bei orthogonaler Ausrichtung die Strahlung zur Strahlenquelle zurück reflektiert wird.

In der US 2007/0183041 A1 ist ein Nachverfolgungssystem mit retroreflektierenden Markern beschrieben. Aus der WO 2013/126361 A1 sind Mikrokügelchen und Transfergegenstände bekannt. Trackersysteme für die optische medizinische Navigation, passive optische Markervorrichtungen für Trackersysteme sowie deren Verwendung sind in der DE 10 2010 036 719 A1 offenbart. Die DE 10 2008 022 254 A1 betrifft Marker für ein Navigationssystem. Ferner sind in der EP 1 639 958 A1 ein rückstrahlender Marker und ein Verfahren zu seiner Herstellung beschrieben.

Es ist daher eine Aufgabe der vorliegenden Erfindung, ein Markerelement, eine Referenzierungseinheit und ein Navigationssystem der eingangs beschriebenen Art so zu verbessern, dass die Bestimmung von Position und/oder Orientierung der Referenzierungseinheit im Raum verbessert wird.

Diese Aufgabe wird bei einem Markerelement der eingangs beschriebenen Art erfindungsgemäß dadurch gelöst, dass der Träger aus einem für elektromagnetische Strahlung durchlässigen Trägermaterial gebildet ist und dass eine äußere Oberfläche des Markerelements durch den Träger gebildet ist, dass das Material, aus dem die retroreflektierenden Elemente ausgebildet sind, einen Brechungsindex mit einem Wert in einem Bereich von etwa 1,5 bis etwa 2,5, oder einen Brechungsindex mit einem Wert in einem Bereich von etwa 2,5 bis etwa 3,4 aufweist.

Die erfindungsgemäß vorgeschlagene Lösung ermöglicht es insbesondere, dass auf das Markerelement auftreffende elektromagnetische Strahlung im Wesentlichen vollständig zurückreflektiert wird, also retroreflektiert. Die elektromagnetische Strahlung wird also im Wesentlichen parallel in die Richtung zurück reflektiert, aus der sie auf das Markerelement und insbesondere dessen retroreflektierende Elemente auftrifft. Zudem ist es nicht zwingend erforderlich, dass alle retroreflektierenden Elemente, insbesondere wenn sie in Form von Kugeln ausgebildet sind, den exakt gleichen Durchmesser aufweisen. Dies spielt insbesondere deshalb keine Rolle, weil jedes einzelne retroreflektierende Element das einfallende Licht wieder in dieselbe Richtung zurückreflektiert. Auf die Größe des retroreflektierenden Elements kommt es dabei nicht an. Für die Herstellung und Handhabung des Markerelements ist es vorteilhaft, dass das Markerelement einen Träger für die Schicht retroreflektierender Elemente umfasst. Der Träger dient insbesondere einerseits dazu, die Mehrzahl retroreflektierender Elemente zu tragen. Zum anderen wirkt er insbesondere auch als Schutzschicht für die retroreflektierenden Elemente. Vorzugsweise ist der Träger aus einem für elektromagnetische Strahlung durchlässigen Trägermaterial gebildet. Insbesondere ist es günstig, wenn das Trägermaterial das Anhaften von Flüssigkeiten und/oder anderen Verschmutzungen möglichst nicht zulässt. So kann verhindert werden, dass praktisch nur ein Teil des Markerelements für das Navigationssystem "sichtbar" ist, wenn Schmutz einen Teil einer äußeren Oberfläche des Markerelements bedeckt. Die Durchlässigkeit des Trägermaterials für elektromagnetische Strahlung ermöglicht es insbesondere, dass die zur Navigation der Referenzierungseinheit eingesetzte elektromagnetische Strahlung den Träger möglichst ungehindert durchdringen, zu den retroreflektierenden Elementen gelangen und von diesen in die Einfallsrichtung oder parallel zu dieser zurückreflektiert werden kann. Insbesondere ist es günstig, dass eine äußere Oberfläche des Markerelements durch den Träger gebildet ist. Der Träger kann so die retroreflektierenden Elemente vor Verschmutzungen schützen, insbesondere vor Verschmutzungen, die sonst in Zwischenräume zwischen die retroreflektierenden Elemente eindringen und dort anhaften könnten. Besonders günstig ist es, dass das Material, aus dem die retroreflektierenden Elemente ausgebildet sind, einen Brechungsindex mit einem Wert in einem Bereich von etwa 1,5 bis etwa 2,5 oder einen Brechungsindex mit einem Wert in einem Bereich von etwa 2,5 bis etwa 3,4 aufweist. Ein Wert des Brechungsindex in den angegebenen Bereichen ist insbesondere dann günstig, wenn, wie nachfolgend noch vorgeschlagen, die retroreflektierenden Elemente mit einer Schutzschicht versehen sind. In diesem Fall ist es vorteilhaft, wenn der Brechungsindex der retroreflektierenden Elemente an den Brechungsindex der Beschichtung angepasst ist, um die gewünschte Retroreflexion trotz vorhandener Schutzschicht zu erreichen.

Auf besonders einfache Weise ausbilden lässt sich das Markerelement, wenn die retroreflektierenden Elemente in Form von Kugeln ausgebildet sind. Kugeln, insbesondere Glaskugeln, lassen sich in großer Stückzahl und hoch reproduzierbar herstellen. Einfallendes Licht wird durch die Kugeln parallel zur Einfallsrichtung zurückreflektiert.

Um insbesondere auch Markerelemente mit kleinen Durchmessern retroreflektierend ausbilden zu können, ist es günstig, wenn die Kugeln einen Durchmesser in einem Bereich von etwa 10 µm bis etwa 50 µm aufweisen. Insbesondere ist es günstig, wenn die Kugeln einen Durchmesser von etwa 20 µm aufweisen.

Vorteilhafterweise sind die retroreflektierenden Elemente aus Glas oder einem Kunststoff hergestellt. Insbesondere Kugeln lassen sich sowohl aus Glas als auch Kunststoff in hoher Qualität und Präzision herstellen.

Besonders günstig ist es, wenn das Material, aus dem die retroreflektierenden Elemente ausgebildet sind, einen Brechungsindex mit einem Wert von etwa 1,93 oder einen Brechungsindex mit einem Wert von etwa 2,9 aufweist.

Besonders kostengünstig und effizient ausbilden lassen sich Markerelemente, wenn die Schicht aus der Mehrzahl retroreflektierender Elemente einlagig gebildet ist. Insbesondere ist es günstig, wenn eine äußere Oberfläche des Markerelements vollständig mit einer einlagigen Schicht aus retroreflektierenden Elementen versehen ist. Auf diese Weise kann die gesamte Oberfläche des Markerelements einen möglichst großen Teil einfallender elektromagnetischer Strahlung in die Richtung zurücksenden, aus der sie auf das Markerelement auftrifft.

Um eine möglichst gute Retroreflexion zu erhalten, ist es vorteilhaft, wenn die Mehrzahl retroreflektierender Elemente jeweils mindestens teilweise mit einer elektromagnetische Strahlung reflektierenden Beschichtung versehen ist. Ähnlich wie bei einem Spiegel können so retroreflektierende Elemente aus Glas, die mit der angegebenen Beschichtung, beispielsweise auf einer Rückseite derselben, versehen sind, einfallende elektromagnetische Strahlung parallel zur Einfallsrichtung zurück reflektieren. Die elektromagnetische Strahlung wird dann insbesondere an der Grenzschicht zwischen dem retroreflektierenden Element und der Beschichtung reflektiert.

Auf besonders einfache Weise herstellen lässt sich das Markerelement, wenn die Beschichtung aus einem Metall gebildet ist. Beispielsweise kann die Beschichtung durch Aufdampfen hergestellt werden.

Besonders gute retroreflektierende Eigenschaften weist ein Markerelement auf, wenn das Metall Silber oder Aluminium ist. Insbesondere Beschichtungen aus Aluminium lassen sich einfach und kostengünstig herstellen.

Besonders einfach und kostengünstig herstellen lässt sich das Markerelement, wenn das Trägermaterial Glas oder Kunststoff ist. Diese Materialien können insbesondere elektromagnetische Strahlung durchlassend ausgebildet sein. Der Einsatz eines Trägermaterials aus Kunststoff hat insbesondere den Vorteil, dass gegebenenfalls auch noch eine Verformung des Trägers nach dem Aufbringen der Schicht retroreflektierender Elemente möglich ist, beispielsweise wenn der Kunststoff ein thermoplastischer Kunststoff ist.

Günstigerweise ist oder enthält der Kunststoff Polymethylmethacrylat (PMMA). Dieser, auch als Acrylglas bezeichnete Kunststoff ist für elektromagnetische Strahlung durchlässig. Zudem kann er auf einfache und kostengünstige Weise hergestellt werden. Die Beschichtung des Trägers aus PMMA mit den retroreflektierenden Elementen kann insbesondere dann erfolgen, wenn der Kunststoff noch nicht vollständig ausgehärtet ist, sodass die retroreflektierenden Elemente auf dessen Oberfläche anhaften oder sogar teilweise in den Träger eingebetter werden können.

Gemäß einer weiteren bevorzugten Ausführungsform der Erfindung kann vorgesehen sein, dass das Trägermaterial einen Trägermaterialbrechungsindex aufweist, welcher kleiner ist als der Brechungsindex der retroreflektierenden Elemente. Dies ermöglicht es insbesondere, dass auf das Markerelement auftreffende elektromagnetische Strahlung durch den Träger hindurch gelangen und auf die retroreflektierenden Elemente auftreffen kann.

Vorzugsweise weist der Trägermaterialbrechungsindex einen Wert in einem Bereich von etwa 1,3 bis etwa 1,7 auf. Insbesondere kann der Trägermaterialbrechungsindex einen Wert von etwa 1,5 aufweisen. Beispielsweise weist der Brechungsindex von PMMA einen Wert von etwa 1,49 auf, so dass dieser Kunststoff hervorragend als Trägermaterial geeignet ist.

Um ein möglichst stabiles Markerelement zu erhalten, ist es vorteilhaft, wenn die Schicht retroreflektierender Elemente mindestens teilweise in den Träger eingebettet ist. So kann eine besonders gute Anhaftung der retroreflektierenden Elemente am Träger erreicht werden.

Die Herstellung des Markerelements wird weiter vereinfacht, wenn die reflektierende Beschichtung auf die mindestens teilweise in den Träger eingebettete Schicht retroreflektierender Elemente aufgebracht ist. Dies kann insbesondere dadurch erreicht werden, dass bei der Herstellung des Markerelements zunächst der Träger mit der Schicht retroreflektierender Elemente versehen wird und erst danach die elektromagnetische Strahlung reflektierende Beschichtung auf die Schicht retroreflektierender Elemente aufgebracht wird.

Markerelemente können grundsätzlich eine beliebige Form aufweisen. Günstig ist es insbesondere, wenn die Schicht retroreflektierender Elemente eben oder im Wesentlichen eben ist oder einen Ausschnitt einer Ellipsenoberfläche oder einer Kugeloberfläche definiert. So können insbesondere ebene, also flache Markerelemente ausgebildet werden oder auch kugelförmige oder im Wesentlichen kugelförmige Markerelemente. Insbesondere kugelförmige Markerelemente haben den Vorteil, dass sie praktisch unabhängig von einer Orientierung im Raum für das Navigationssystem stets eine im Wesentlichen kreisförmige Ansichtsfläche zeigen.

Vorzugsweise ist das Markerelement ellipsenförmig oder kugelförmig oder im Wesentlichen kugelförmig ausgebildet. Wie bereits dargelegt, spielt es so für die Navigation der Referenzierungseinheit praktisch keine Rolle, wie das Markerelement im Raum orientiert ist. Das Navigationssystem sieht so im Wesentlichen stets eine kreisförmige Fläche.

Gemäß einer weiteren bevorzugten Ausführungsform der Erfindung kann vorgesehen sein, dass die reflektierende Beschichtung in Richtung oder im Wesentlichen in Richtung auf einen Mittelpunkt des kugelförmigen oder im Wesentlichen kugelförmigen Markerelements hin weist. Dies bedeutet insbesondere, dass der Träger vom Mittelpunkt des Markerelements weg weist und so eine äußere, die retroreflektierenden Elemente schützende Schicht oder Beschichtung bildet.

Für die Herstellung des Markerelements kann es von Vorteil sein, wenn es zwei- oder mehrteilig ausgebildet ist. Insbesondere kann es aus zwei oder mehr Markerelementteilen bestehen. Beispielsweise lässt sich so ein kugelförmiges Markerelement auf einfache Weise herstellen.

Günstig ist es, wenn die zwei oder mehr Markerelementteile halbschalenförmig oder im Wesentlichen halbschalenförmig ausgebildet sind. So lassen sich insbesondere kugelförmige Markerelemente aus zwei halbschalenförmigen Markerelementteilen zusammensetzen. Beispielsweise können ebene oder halbschalenförmige Träger ausgebildet werden, deren Innenflächen mit den retroreflektierenden Markerelementen beschichtet werden. Zum Ausbilden des Markerelements werden die Markerelementteile dann zusammengefügt.

Vorzugsweise begrenzt die retroreflektierende Beschichtung einen ellipsoiden oder kugeligen oder im Wesentlichen kugeligen Hohlraum. So lassen sich beispielsweise sehr leichte Markerelemente ausbilden. Insbesondere durch die elektromagnetische Strahlung reflektierende Beschichtung, die in Richtung auf einen Mittelpunkt des Hohlraums hinweisen kann, ist es nicht zwingend erforderlich, diesen zu füllen.

Eine Stabilität des Markerelements kann insbesondere dadurch verbessert werden, dass der Hohlraum mit einem Füllmaterial gefüllt ist.

Besonders einfach und kostengünstig ausbilden lässt sich das Markerelement, wenn das Füllmaterial ein Kunststoff ist. Insbesondere kann es sich bei dem Kunststoff um einen sterilisierbaren Kunststoff handeln. Beispielsweise kann das Füllmaterial bereits in einer Form hergestellt werden, die einer Form oder Gestalt des Hohlraums entspricht, sodass insbesondere dann, wenn das Markerelement mehrere Markerelementteile umfasst, diese über das Füllmaterial gestülpt werden können. Das Füllmaterial kann jedoch auch erst beim Zusammenfügen der Markerelementteile in den Hohlraum eingefüllt werden.

Die eingangs gestellte Aufgabe wird ferner bei einer Referenzierungseinheit der eingangs beschriebenen Art erfindungsgemäß dadurch gelöst, dass das mindestens eine Markerelement in Form eines der oben beschriebenen vorteilhaften Markerelemente ausgebildet ist.

Die Referenzierungseinheit mit erfindungsgemäßen Markerelementen auszustatten ermöglicht insbesondere eine verbesserte Sichtbarkeit der Referenzierungseinheit im Raum und damit eine präzisiere Bestimmung von Position und/oder Orientierung derselben im Raum durch das Navigationssystem.

Für die Handhabbarkeit der Referenzierungseinheit ist es günstig, wenn sie einen Träger umfasst, auf welchem das mindestens eine Markerelement angeordnet oder ausgebildet ist. Beispielweise können hier herkömmliche Träger zum Einsatz kommen mit Kopplungseinrichtungen zum lösbaren Verbinden mit jeweils einem Markerelement. Die Markerelemente können dann beispielsweise als Einweg-Markerelemente ausgebildet werden, der Träger kann gereinigt und nach einer Sterilisierung wiederholt zum Einsatz kommen. Selbstverständlich können die Markerelemente auch aufbereitbar ausgebildet sein, um mehrfach verwendet zu werden. Voraussetzung ist dann lediglich, dass die Materialien, aus denen die Markerelemente ausgebildet sind, insbesondere resistent gegen alkalische Reinigungsmedien sind sowie das Durchlaufen mindestens eines Heißdampfsterilisationszyklus aushalten.

Des Weiteren wird die eingangsgestellte Aufgabe bei einem Navigationssystem der eingangs beschriebenen Art erfindungsgemäß dadurch gelöst, dass das mindestens eine Markerelement eines der oben beschriebenen vorteilhaften Markerelemente ist und/oder dass die Referenzierungseinheit in Form einer der oben beschriebenen vorteilhaften Referenzierungseinheiten ausgebildet ist.

Ein Navigationssystem mit solchen Markerelementen beziehungsweise Referenzierungseinheiten auszustatten hat den Vorteil, dass die Sichtbarkeit sowohl der Markerelemente als auch der Referenzierungseinheiten durch die Nachweisvorrichtung des Navigationssystems im Vergleich zu herkömmlichen Markerelementen mit diffus reflektierenden Oberflächen deutlich verbessert ist.

Die nachfolgende Beschreibung einer bevorzugten Ausführungsform der Erfindung dient im Zusammenhang mit der Zeichnung der näheren Erläuterung. Es zeigen:
- Figur 1:: eine schematische Darstellung eines Navigationssystems;

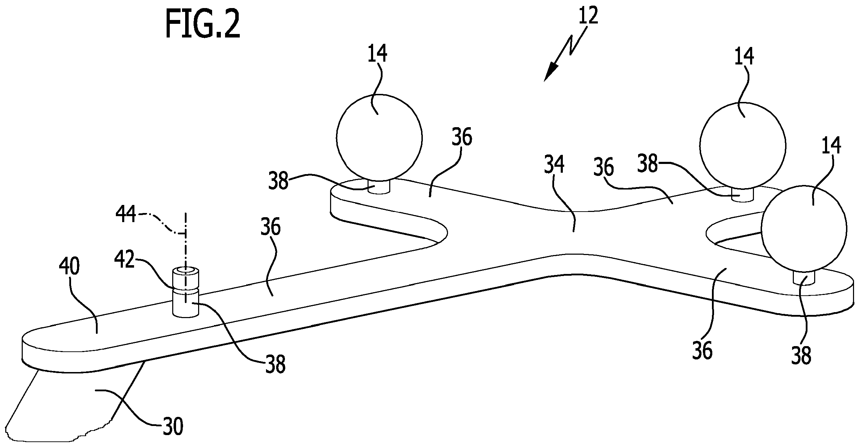

- Figur 2:: eine perspektivische Darstellung einer Referenzierungseinheit mit vier Markerelementen, von denen eines in einer Schnittansicht dargestellt ist;
- Figur 3:: eine schematische Darstellung des Grundprinzips der Retroreflexion an einer Kugel;
- Figur 4:: eine schematische Darstellung des Strahlengangs durch einen aus PMMA hergestellten, mit einer Schicht retroreflektierender Elemente versehenen Träger;
- Figur 5:: eine schematische Ansicht eines hohlkugelförmigen Markerelements mit einer Schicht retroreflektierender Elemente, welche auf einer inneren Wandfläche einer hohlkugelförmigen äußere Schutzschicht angeordnet sind;
- Figur 6:: eine schematische Darstellung des Strahlengangs beim Einsatz eines Markerelements aus Figur 5;
- Figur 7:: eine schematische Schnittansicht eines zweiteiligen Markerelements mit einer äußeren Schutz-/Trägerschicht;
- Figur 8:: eine schematische Darstellung des Strahlengangs ähnlich Figur 6, jedoch bei einem Markerelement wie in Figur 7 dargestellt; und
- Figur 9:: eine schematische Darstellung einer Intensitätsverteilung der von dem in Figur 5 dargestellten kugelförmigen Markerelement retroreflektierten Strahlung.

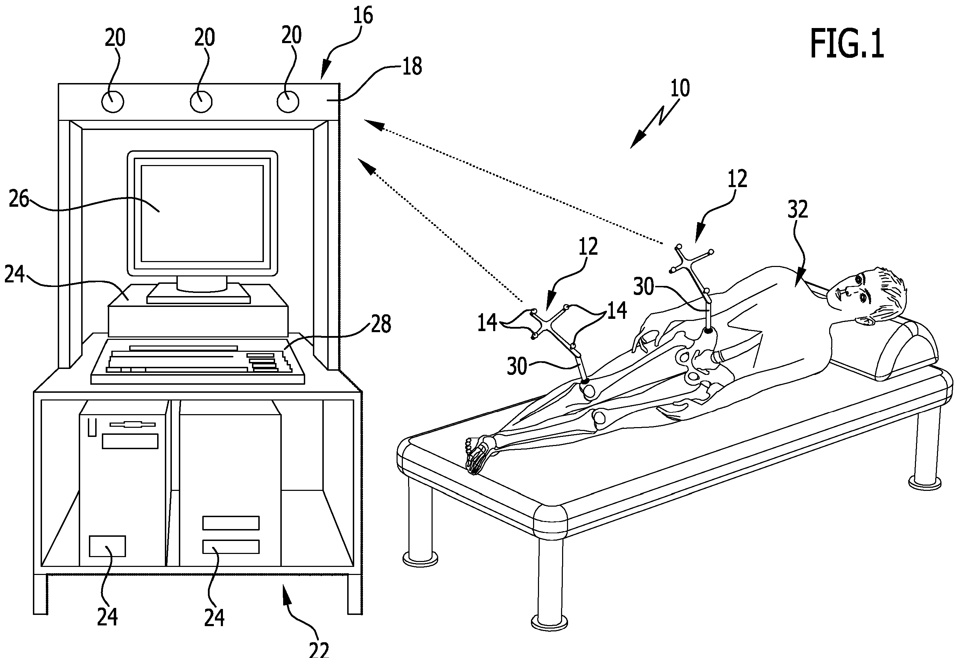

In Figur 1 ist beispielhaft ein insgesamt mit dem Bezugszeichen 10 versehenes Navigationssystem dargestellt. Es umfasst mehrere Referenzierungseinheiten 12, welche vorzugsweise mindestens drei, bei den in den Figuren dargestellten Ausführungsbeispielen jeweils vier, Markerelemente 14 umfassen.

Das Navigationssystem 10 umfasst eine Sende- und Empfangseinheit 16 zum Aussenden und Empfangen von elektromagnetischer Strahlung und/oder Ultraschall. Sie umfasst einen balkenförmigen Träger 18, an welchem drei Sender/Empfänger 20 angeordnet sind, mit welchen elektromagnetische Strahlung beziehungsweise Ultraschall ausgesandt und/oder empfangen werden können. Grundsätzlich könnten nur zwei Sender/Empfänger 20 vorgesehen sein. Um eine Genauigkeit bei der Positionsbestimmung der Referenzierungseinheiten 12 zu verbessern, können auch drei oder mehr derartige Sender/Empfänger 20 vorgesehen werden. Des Weiteren umfasst das Navigationssystem 10 eine Datenverarbeitungsanlage 22, welche bei dem in Figur 1 dargestellten Ausführungsbeispiel drei miteinander zusammengeschaltete Computer 24, einen Monitor 26 und ein Eingabegerät in Form einer Tastatur 28 umfasst. Mit der Datenverarbeitungsanlage 22 können von der Sende- und Empfangseinheit 16 erzeugte und/oder empfangene Signale verarbeitet werden, um eine Position und/oder eine Orientierung einer Referenzierungseinheit 12 im Raum zu bestimmen.

Referenzierungseinheiten 12 können insbesondere derart ausgebildet sein, dass sie mit entsprechenden Adaptern 30 zum Beispiel an einem Patienten 32 festgelegt werden können. Insbesondere können so Gelenkpositionen und Gelenkzentren des Patienten 32 ermittelt werden, indem ein Körperteil des Patienten 32, an dem ein erstes Referenzelement 12 festgelegt ist, relativ zu einem anderen Körperteil des Patienten 32 bewegt wird, an welchem eine weitere Referenzierungseinheit 12 festgelegt ist. Alternativ kann eine Referenzierungseinheit 12 auch an einem chirurgischen Instrument oder einem Werkzeug angeordnet sein, zum Beispiel unter Verwendung eines dafür geeigneten Adapters 30.

Die Referenzierungseinheit 12 umfasst einen kreuzförmigen Träger 34, welcher vier im Wesentlichen senkrecht relativ zueinander angeordnete Trägerarme 36 umfasst, die insbesondere unterschiedliche Längen aufweisen können. Jeder Trägerarm 36 trägt ein Verbindungselement in Form eines zapfenförmigen Adapters 38, welche jeweils senkrecht von einer ebenen Oberfläche 40 des Trägers 34 abstehen. Jeder Adapter 38 ist mit einer umlaufenden Ringnut 42 versehen, welche ein Rastelement bildet. Die Ringnut 42 ist konzentrisch zu einer vom Adapter 38 definierten Längsachse 44 angeordnet und teilt den Adapter 38 in zwei Teile, die in einem Längenverhältnis von etwa zwei zu drei stehen, wobei das längere Teil des Adapters 38 direkt an den Träger 34 angrenzt.

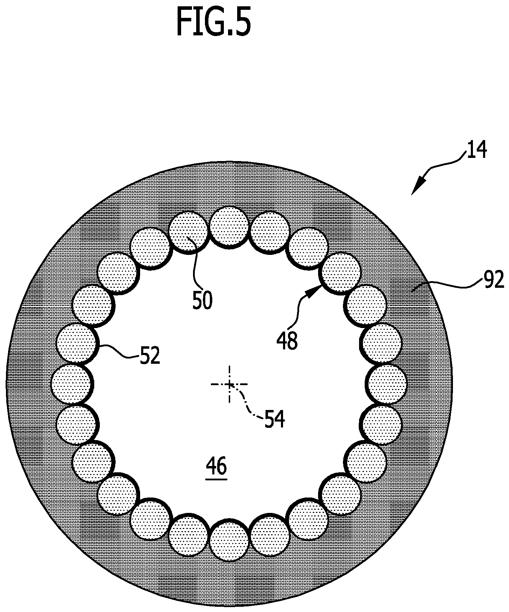

In Figur 5 ist schematisch der Aufbau eines ersten Ausführungsbeispiels eines der Markerelemente 14 dargestellt. Es umfasst einen in Form einer Kugelschale ausgebildeten Träger 92, welcher einen hohlen Kern 46 umgibt. Der Träger 92 ist auf einer einem Mittelpunkt 54 der Kugelschale zugewandten Innenseite mit einer Mehrzahl retroreflektierender Elemente 48 in Form von Kugeln 50 aus Kunststoff oder Glas versehen ist.

Eine äußere Oberfläche der Kugeln 50 ist teilweise mit einer elektromagnetische Strahlung reflektierenden Beschichtung 52 versehen. Die Beschichtung 52 bedeckt die Hälfte der äußeren Oberfläche jeder Kugel 50, und zwar derart, dass die äußeren Oberflächen der Kugeln 50 im Wesentlichen nur auf ihrer an den hohlen Kern angrenzenden Oberflächenbereichen mit der Beschichtung 52 versehen sind. Die Kugeln 50 mit der Beschichtung 52 sind also derart angeordnet, dass die beschichteten, halbkugeligen Oberflächen in Richtung auf einen Mittelpunkt 54 des Kerns 46 hin weisen.

Die retroreflektierenden Elemente 48 können direkt auf die Innenseite beziehungsweise auf die innere Oberfläche des Trägers 92 mit einem Klebstoff oder Haftmittel aufgebracht sein. Optional können sie auch teilweise in den Träger 92 eingebettet sein.

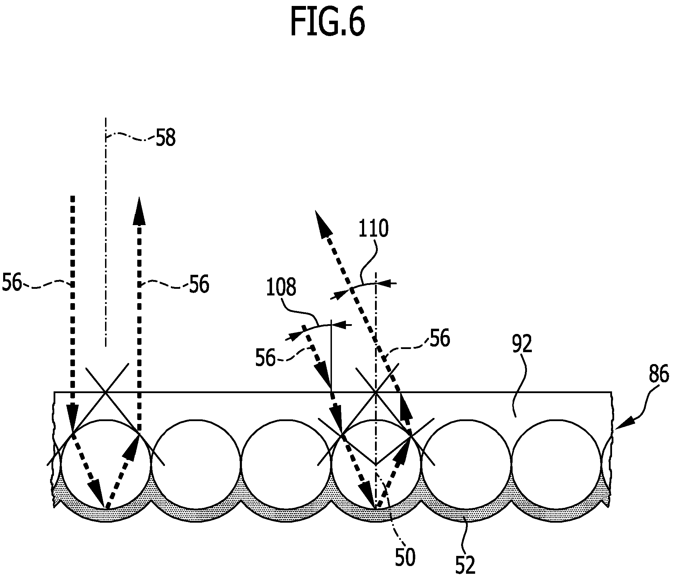

In Figur 6 ist schematisch ein Strahlengang von auf das Markerelement 14 auftreffender elektromagnetischer Strahlung 56 dargestellt. Hier findet ein Übergang von einem optisch dünneren zu einem optisch dichteren Medium bereits beim Auftreffen der Strahlung auf den Träger 92 statt. Trifft die Strahlung 56 nicht senkrecht auf den Träger 92, dann wird sie bereits beim Übergang von Luft in den Träger 92 zum Lot hin gebrochen.

Der Brechungsindex der Kugeln 50 ist vorzugsweise so gewählt, dass er größer ist als ein Brechungsindex des Trägermaterials, aus dem der Träger 92 hergestellt ist. Beispielsweise dann, wenn der Träger 92 aus Polymethylmethacrylat (PMMA) gebildet ist, dessen Brechungsindex etwa 1,49 beträgt, ist es vorteilhaft, wenn ein Brechungsindex der Kugeln 50 etwa 2,9 beträgt. Dies kann durch entsprechende Auswahl eines Kunststoffes oder eines Glases zur Ausbildung der Kugeln 50 erreicht werden. Durch diese Wahl der Brechungsindizes ergibt sich ein weitere Übergang von einem optisch dünneren Medium, nämlich dem Träger 92, in ein optisch dichteres Medium, nämlich die Kugeln 50. Wiederum wird die Strahlung 56 zum Lot hin gebrochen.

Jede Kugel 50 kann mehrschichtig ausgebildet sein oder auch aus einer Materialmischung bestehen. Diese Ausgestaltungen ermöglichen insbesondere eine individuelle Anpassung des Brechungsindex der Kugeln 50.

An der Grenzschicht zwischen der Kugel 50 und der Beschichtung 52 wird die Strahlung 56 reflektiert. Ferner ändert die Strahlung 56 nochmals beim Austritt aus der Kugel 50 in den Träger 92 sowie beim Austritt aus dem Träger 92 in die umgebende Luft ihre Richtung, so dass sie parallel zu einer Einfallsrichtung 58, zu der sie ebenfalls parallel auf die Kugel 50 aufgetroffen ist, wieder zum Sender/Empfänger 20 des Navigationssystems 10 zurückgesandt wird.

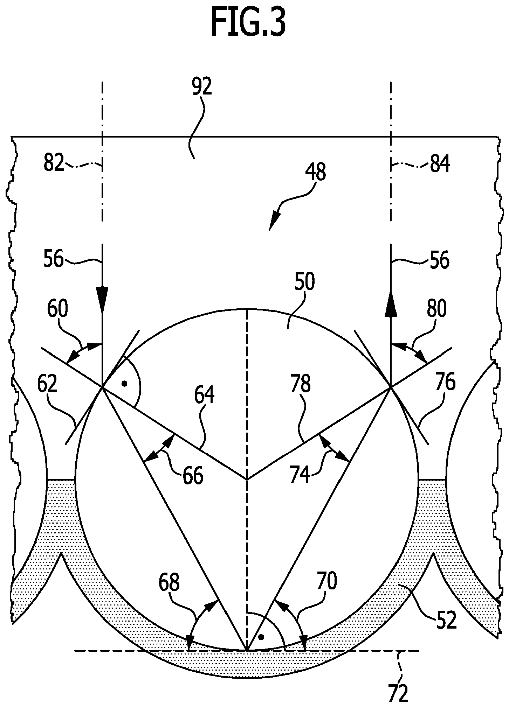

In Figur 3 ist der Strahlengang aus Figur 6 nochmals im Detail an einer Kugel 50 dargestellt. Die senkrecht auf den Träger 92 auftreffende Strahlung 56 trifft unter einem Einfallswinkel 60 bezogen auf eine Tangentialebene 62 der Kugel 50 auf die Kugel 50 auf und wird zu der zur Tangentialebene 62 senkrecht stehenden Flächennormalen 64 hin gebrochen. Ein Ausfallswinkel 66 im optisch dichteren Medium ist kleiner als der Einfallswinkel 60.

An einer Grenzschicht zwischen der Kugel 50 und der Beschichtung 52 wird die Strahlung 56 total reflektiert. Hier gilt, dass ein Einfallswinkel 68 mit dem Ausfallswinkel 70 übereinstimmt, wobei die Reflexion bezogen auf eine Tangentialebene 72 an der Grenzschicht zwischen Kugel 50 und Beschichtung 52 erfolgt.

Da die Kugel 50 vollständig symmetrisch ist, trifft die Strahlung 56 unter einem Ausfallswinkel 74, welcher mit dem Ausfallswinkel 66 übereinstimmt, wieder auf eine Grenzfläche zwischen der Kugel 50 und dem Träger. Bezogen auf eine senkrecht zu einer Tangentialebene 76 im Austrittspunkt der Strahlung 56 aus der Kugel 50 verlaufende Flächennormale 78 tritt die Strahlung 56 unter einem Einfallswinkel 80 aus der Kugel 50 aus, wobei der Einfallswinkel 80 dem Einfallswinkel 60 entspricht. Eine Einfallsrichtung 82 der Strahlung 56 verläuft somit parallel zu einer Ausfallsrichtung 84 der total reflektierten Strahlung 56.

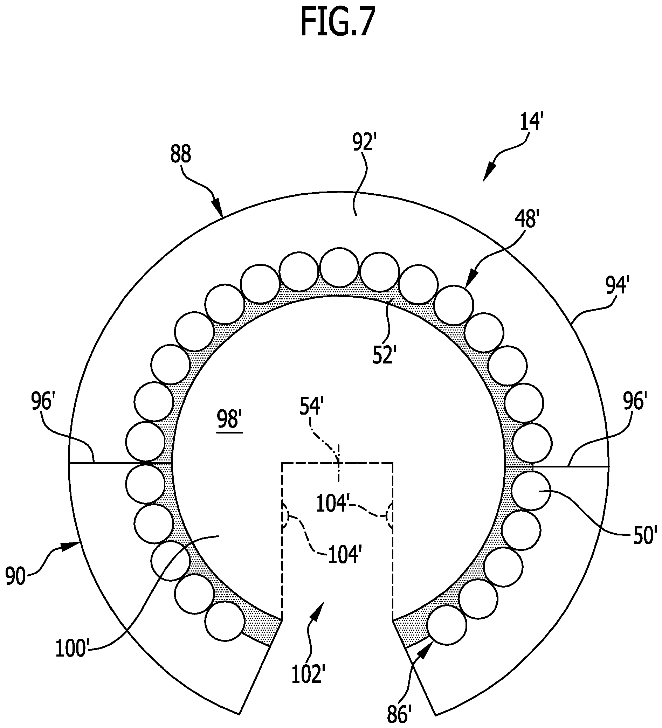

In Figur 7 ist ein zweites Ausführungsbeispiel eines insgesamt mit dem Bezugszeichen 14' bezeichneten Markerelements schematisch dargestellt. Ähnlich wie beim in Figur 5 dargestellten Markerelement 14 stimmen die Größenverhältnisse zwischen den retroreflektierenden Elementen 48 beziehungsweise 48' nicht mit den Dimensionen der Markerelemente 14 und 14' überein. Die Markerelemente 14 und 14' können beispielsweise einen Durchmesser von etwa 10 mm aufweisen, die retroreflektierenden Elemente 48 und 48' können einen Durchmesser von etwa 20 µm aufweisen. Daraus ergibt sich, dass eine Schicht 86 beziehungsweise 86' der retroreflektierenden Elemente 48 beziehungsweise 48' deutlich mehr Kugeln 50 beziehungsweise 50' umfasst als in den Figuren dargestellt ist.

Das Markerelement 14' ist zweiteilig ausgebildet und umfasst ein erstes Markerelementteil 88' und ein zweites Markerelementteil 90', die jeweils im Wesentlichen halbschalig ausgebildet sind. Jedes Markerelementteil 88' umfasst einen eine elektromagnetische Strahlung durchlässigen Träger 92', welche eine äußere Oberfläche 94' aufweist, die von einem Mittelpunkt 54' des Markerelements 14' weg weist. Die Oberfläche 94' ist somit vom Markerelement 14' weg weisend konvex gekrümmt.

Die Kugeln 50' sind teilweise in den Träger 92' eingebettet. Bei dem in Figur 7 dargestellten Ausführungsbeispiel des Markerelements 14' etwa zur Hälfte.

Die Schicht 86' der retroreflektierenden Elemente 48' ist einlagig ausgebildet und weist mit ihrer vom Träger 92' weg weisenden Seiten Richtung auf den Mittelpunkt 54'. Ferner sind die Kugeln 50' wiederum mit einer elektromagnetische Strahlung reflektierenden Beschichtung 52' versehen. Diese bildet eine innere Oberfläche der Halbkugeln definierenden Markerelementteile 88' und 90'. Die Markerelementteile 88' und 90' sind stoffschlüssig mit einer Klebstoffschicht 96' miteinander verbunden.

Optional kann ein von der Beschichtung 52' begrenzter kugeliger Hohlraum 98' mit einem Füllmaterial ausgefüllt sein. Dieses bildet vorzugsweise die Form einer Kugel 100' mit einer Kupplungsausnehmung 102', welche vorzugsweise korrespondierend zum Adapter 38 ausgebildet ist.

An der Kupplungsausnehmung 102' können insbesondere vorspringende Rastelemente 104' vorgesehen sein, die in die Ringnut 42 eingreifen, wenn das Markerelement 14' mit dem Adapter 38 gekoppelt ist.

Die Kugel 100' ist vorzugsweise aus einem sterilisierbaren Kunststoff gebildet. Insbesondere können die Markerelementteile 88' und 90' stoffschlüssig mit der Kugel 100' verbunden sein, beispielsweise mittels einer Klebstoffschicht.

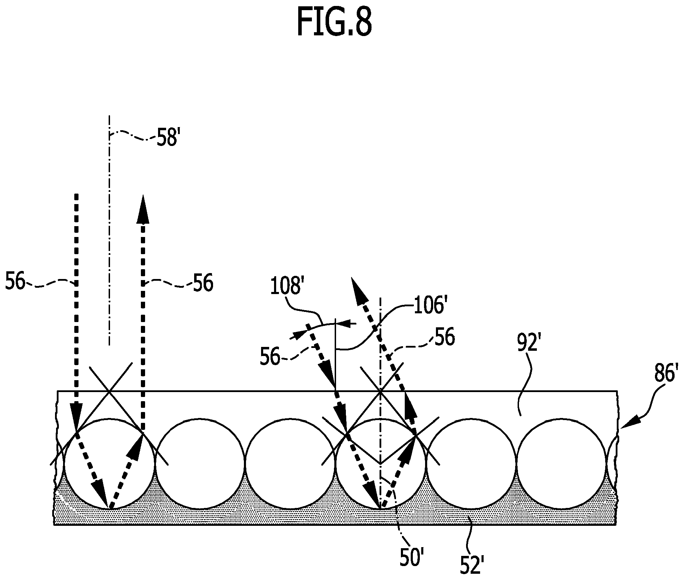

In Figur 8 ist schematisch ein Strahlengang von auf das Markerelement 14' auftreffender elektromagnetischer Strahlung 56 dargestellt. Hier findet ein Übergang von einem optisch dünneren zu einem optisch dichteren Medium bereits beim Auftreffen der Strahlung auf den Träger 92' statt.

Der Brechungsindex der Kugeln 50' ist vorzugsweise so gewählt, dass er größer ist als ein Brechungsindex des Trägermaterials, aus dem der Träger 92' hergestellt ist. Beispielsweise dann, wenn der Träger 92' aus Polymethylmethacrylat (PMMA) gebildet ist, dessen Brechungsindex etwa 1,49 beträgt, ist es vorteilhaft, wenn ein Brechungsindex der Kugeln 50' etwa 2,9 beträgt. Dies kann durch entsprechende Auswahl eines Kunststoffes oder eines Glases zur Ausbildung der Kugeln 50' erreicht werden.

Jede Kugel 50' kann, ebenso wie jede Kugel 50, mehrschichtig ausgebildet sein oder auch aus einer Materialmischung bestehen. Diese Ausgestaltungen ermöglichen insbesondere eine individuelle Anpassung des Brechungsindex der Kugeln 50 und 50'.

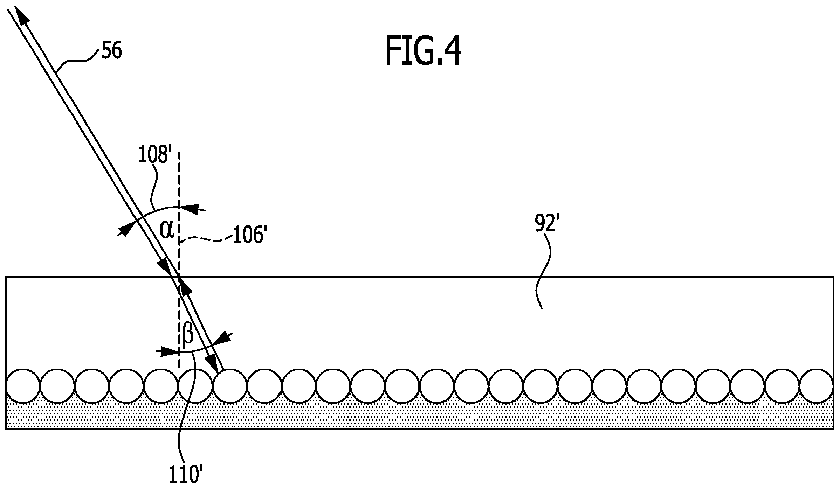

Wie in den Figuren 4 und 8 zu erkennen ist, wird die elektromagnetische Strahlung 56 beim Eintritt in den Träger 92' zur Flächennormalen 106' einer äußeren Oberfläche des Trägers 92' hin gebrochen. Ein Einfallswinkel 108' ist größer als ein Ausfallswinkel 110' im optisch dichteren Träger 92'. Der Strahlengang der elektromagnetischen Strahlung 56 beim Übergang vom Träger 92' in die Kugel 50' entspricht im Wesentlichen dem Verlauf wie in Figur 3 dargestellt, denn der Träger 92' ist aus einem optisch dünneren Material hergestellt als die Kugeln 50' so dass das im Zusammenhang mit Figur 3 erläuterte Brechungsgesetz hier analog Anwendung findet.

Der Träger 92' ermöglicht eine möglichst störungsfreie Retroreflektion, denn eine Verschmutzung einer äußeren Oberfläche des Trägers 92', beispielsweise durch Fett oder Wasser, führt dann lediglich zu einem Parallelversatz und nicht zu einer massiven Störung der Sichtbarkeit des Markerelements 14', wie dies bei herkömmlichen Markerelementen der Fall ist.

Die Herstellung der Markerelementteile 88 und 90' kann erfolgen, indem zunächst ein ebener Träger 92' mit der Schicht 86' der retroreflektierenden Elemente 48' versehen wird. Eine so beispielsweise mit eingebetteten Kugeln 50' versehene Platte aus Acrylglas kann dann in einem nächsten Schritt zu einer halben Hohlkugel geformt werden, wie sie im Schnitt in Figur 7 als Markerelementteil 88' beziehungsweise 90' beispielhaft dargestellt ist.

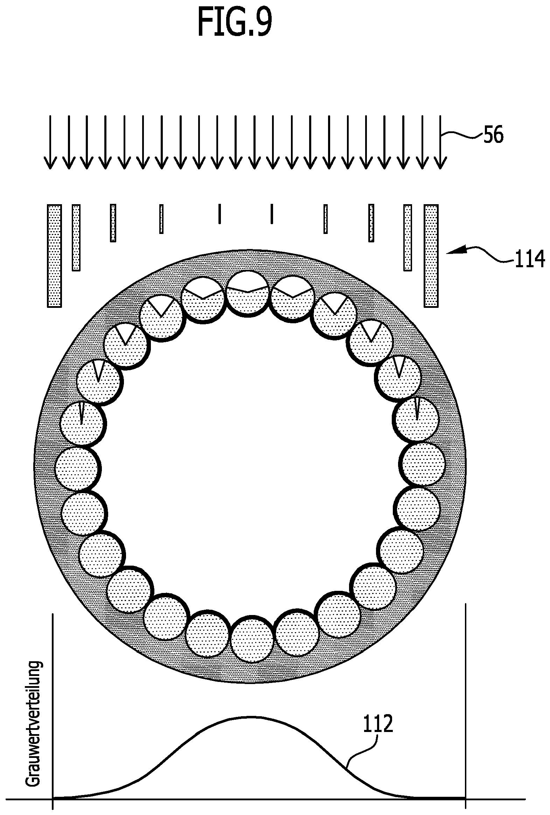

In Figur 9 ist schematisch eine Intensitätsverteilung der von einem Markerelement 14 beziehungsweise 14' retroreflektierten Strahlung dargestellt, wie sie sich aus einem mit der Sende- und Empfangseinheit 16 des Navigationssystems 10 aufgenommenen Bild des Markerelements 14 beziehungsweise 14' ergibt. Da nur ein Teil der Strahlung 56 von den retroreflektierenden Elementen 48 beziehungsweise 48' bei Einfall von Strahlung 56 auf das Markerelement 14 beziehungsweise 14' unter Berücksichtigung der Brechungsindizes der Kugeln 50 beziehungsweise 50' sowie der Trägers 92 beziehungsweise 92' retroreflektiert werden kann, ergibt sich im äußeren Bereich des Bildes der Markerelemente 14 beziehungsweise 14' ein deutlich abgeschwächtes Intensitätssignal. Die nicht reflektierten Anteile 114 der Strahlung 56 sind oberhalb des schematisch dargestellten Markerelements beispielhaft als punktierte Balken symbolisiert.

Insgesamt erhält man mit den Markerelementen 14 und 14' eine Intensitäts- oder Grauwertverteilung mit einem Maximum, welches bei einem unverschmutzten Markerelement 14 beziehungsweise 14' einen Mittelpunkt des Markerelements 14 beziehungsweise 14' angibt. Dies liegt daran, dass bei der Ausbildung der Markerelemente 14 beziehungsweise 14' in der beschriebenen Weise jeweils nur ein Teil der Kugel 50 beziehungsweise 50' die einfallende Strahlung überhaupt zurücksenden kann.

### Bezugszeichenliste

- 10: Navigationssystem
- 12: Referenzierungseinheit
- 14: Markerelement
- 16: Sende- und Empfangseinheit
- 18: Träger
- 20: Sender/Empfänger
- 22: Datenverarbeitungsanlage
- 24: Computer
- 26: Monitor
- 28: Tastatur
- 30: Adapter
- 32: Patient
- 34: Träger
- 36: Trägerarm
- 38: Adapter
- 40: Oberfläche
- 42: Ringnut
- 44: Längsachse
- 46: Kern
- 48, 48': Retroreflektierendes Element
- 50, 50': Kugel
- 52, 52': Beschichtung
- 54, 54': Mittelpunkt
- 56: Strahlung
- 58, 58': Einfallsrichtung
- 60: Einfallswinkel
- 62: Tangentialebene
- 64: Flächennormale
- 66: Ausfallswinkel
- 68: Einfallswinkel
- 70: Ausfallswinkel
- 72: Tangentialebene
- 74: Ausfallswinkel
- 76: Tangentialebene
- 78: Flächennormale
- 80: Einfallswinkel
- 82: Einfallsrichtung
- 84: Ausfallsrichtung
- 86, 86': Schicht
- 88': Markerelementteil
- 90': Markerelementteil
- 92, 92': Träger
- 94': Oberfläche
- 96': Klebstoffschicht
- 98': Hohlraum
- 100': Kugel
- 102': Kupplungsausnehmung
- 104': Rastelemente
- 106': Flächennormale
- 108, 108': Einfallswinkel
- 110, 110': Ausfallswinkel
- 112: Intensitätsverteilung
- 114: nicht reflektierte Anteile

## Patentansprüche

1. Markerelement (14; 14'), insbesondere medizinisches oder chirurgisches Markerelement (14; 14'), für eine Referenzierungseinheit (12) eines Navigationssystems (10), welches Markerelement (14; 14') elektromagnetische Strahlung (56) reflektierend ausgebildet ist, wobei das Markerelement eine Schicht (86; 86') umfassend eine Mehrzahl retroreflektierender Elemente (48; 48') und einen Träger (92; 92') für die Schicht (86; 86') retroreflektierender Elemente (48; 48') umfasst, **dadurch gekennzeichnet, dass** der Träger (92; 92') aus einem für elektromagnetische Strahlung (56) durchlässigen Trägermaterial gebildet ist, dass eine äußere Oberfläche des Markerelements (14') durch den Träger (92') gebildet ist und dass das Material, aus dem die retroreflektierenden Elemente (48; 48') ausgebildet sind, einen Brechungsindex mit einem Wert in einem Bereich von etwa 1,5 bis etwa 2,5 oder einen Brechungsindex mit einem Wert in einem Bereich von etwa 2,5 bis etwa 3,4 aufweist.

2. Markerelement nach Anspruch 1, **dadurch gekennzeichnet, dass** die retroreflektierenden Elemente (48; 48') in Form von Kugeln (50; 50') ausgebildet sind,
wobei insbesondere die Kugeln (50; 50') einen Durchmesser in einem Bereich von etwa 10 µm bis etwa 50 µm aufweisen, insbesondere einen Durchmesser von etwa 20 µm.

3. Markerelement nach einem der voranstehenden Ansprüche, **dadurch gekennzeichnet, dass**
a) die retroreflektierenden Elemente (48; 48') aus Glas oder einem Kunststoff hergestellt sind
und/oder
b) die Schicht (86; 86') aus der Mehrzahl retroreflektierender Elemente (48; 48') einlagig gebildet ist.

4. Markerelement nach einem der voranstehenden Ansprüche, **dadurch gekennzeichnet, dass** das Material, aus dem die retroreflektierenden Elemente (48; 48') ausgebildet sind, einen Brechungsindex mit einem Wert von etwa 1,93 oder mit einem Wert von etwa 2,9 aufweist.

5. Markerelement nach einem der voranstehenden Ansprüche, **dadurch gekennzeichnet, dass** die Mehrzahl retroreflektierender Elemente (48; 48') jeweils mindestens teilweise mit einer elektromagnetische Strahlung (56) reflektierenden Beschichtung (52; 52') versehen ist.

6. Markerelement nach Anspruch 5, **dadurch gekennzeichnet, dass**
a) die Beschichtung (52; 52') aus einem Metall gebildet ist, insbesondere durch Aufdampfen,
wobei insbesondere das Metall Silber oder Aluminium ist
und/oder
b) die reflektierende Beschichtung (52') einen ellipsoiden oder kugeligen oder im Wesentlichen kugeligen Hohlraum (98') begrenzt.

7. Markerelement nach einem der Ansprüche 5 oder 6, **dadurch gekennzeichnet, dass**
a) die Schicht (86; 86') retroreflektierender Elemente mindestens teilweise in den Träger (92; 92') eingebettet ist
und/oder
b) die reflektierende Beschichtung (52') auf die mindestens teilweise in den Träger (92') eingebettete Schicht (86') retroreflektierender Elemente (48') aufgebracht ist.

8. Markerelement nach einem der voranstehenden Ansprüche, **dadurch gekennzeichnet, dass** das Trägermaterial Glas oder Kunststoff ist, wobei insbesondere der Kunststoff Polymethylmethacrylat (PMMA) ist oder enthält.

9. Markerelement nach einem der voranstehenden Ansprüche, **dadurch gekennzeichnet, dass** das Trägermaterial einen Trägermaterialbrechungsindex aufweist, welcher kleiner ist als der Brechungsindex der retroreflektierenden Elemente (48; 48'),
wobei insbesondere der Trägermaterialbrechungsindex einen Wert in einem Bereich von etwa 1,3 bis etwa 1,7 aufweist, insbesondere einen Wert von etwa 1,5.

10. Markerelement nach einem der voranstehenden Ansprüche, **dadurch gekennzeichnet, dass**
a) die Schicht (86; 86') retroreflektierender Elemente (48; 48') eben oder im Wesentlichen eben ist oder einen Ausschnitt einer Ellipsenoberfläche oder einer Kugeloberfläche definiert
und/oder
b) das Markerelement (14; 14') ellipsenförmig oder kugelförmig oder im Wesentlichen kugelförmig ausgebildet ist,
wobei insbesondere die reflektierende Beschichtung (52') in Richtung oder im Wesentlichen in Richtung auf einen Mittelpunkt (54') des kugelförmigen oder im Wesentlichen kugelförmigen Markerelements (14') hin weist.

11. Markerelement nach einem der voranstehenden Ansprüche, **dadurch gekennzeichnet, dass** das Markerelement (14') zwei- oder mehrteilig ausgebildet ist, insbesondere aus zwei oder mehr Markerelementteilen (88', 90'),
wobei insbesondere die zwei oder mehr Markerelementteile (88', 90') halbschalenförmig oder im Wesentlichen halbschalenförmig ausgebildet sind.

12. Markerelement nach einem der voranstehenden Ansprüche, **dadurch gekennzeichnet, dass** die reflektierende Beschichtung (52') einen ellipsoiden oder kugeligen oder im Wesentlichen kugeligen Hohlraum (98') begrenzt und dass der Hohlraum (98') mit einem Füllmaterial gefüllt ist, wobei insbesondere das Füllmaterial ein Kunststoff ist, weiter insbesondere ein sterilisierbarer Kunststoff.

13. Referenzierungseinheit (12), insbesondere medizinische oder chirurgische Referenzierungseinheit (12), deren Position und/oder Orientierung im Raum mit einem chirurgischen Navigationssystem (10) detektierbar ist, mit mindestens einem chirurgischen Markerelement (14; 14'), **dadurch gekennzeichnet, dass** das mindestens eine Markerelement (14; 14') in Form eines Markerelements (14; 14') nach einem der voranstehenden Ansprüche ausgebildet ist.

14. Referenzierungseinheit (12) nach Anspruch 13, **gekennzeichnet durch** einen Träger (18), auf welchem das mindestens eine Markerelement (14; 14') angeordnet oder ausgebildet ist.

15. Navigationssystem (10), insbesondere medizinisches oder chirurgisches Navigationssystem (10), mit mindestens einer mindestens drei Markerelemente (14; 14') umfassenden Referenzierungseinheit (12) und mit mindestens einer Nachweisvorrichtung (16) zum Detektieren der Position und/oder der Orientierung der Referenzierungseinheit (12) im Raum, wobei die Referenzierungseinheit (12) mindestens ein chirurgisches Markerelement (14; 14') umfasst, **dadurch gekennzeichnet, dass** mindestens ein Markerelement (14; 14') ein Markerelement (14; 14') nach einem der Ansprüche 1 bis 12 ist und/oder dass die Referenzierungseinheit (12) in Form einer Referenzierungseinheit (12) nach Anspruch 13 oder 14 ausgebildet ist.

## Claims

1. Marker element (14; 14'), in particular a medical or surgical marker element (14; 14'), for a referencing unit (12) of a navigation system (10), which marker element (14; 14') is configured to be reflective to electromagnetic radiation (56), wherein the marker element comprises a layer (86; 86') comprising a multitude of retroreflective elements (48; 48') and a support (92; 92') for the layer (86; 86') of retroreflective elements (48; 48'), **characterized in that** the support (92; 92') is formed out of a support material permeable to electromagnetic radiation (56), **in that** an outer surface of the marker element (14') is formed by the support (92') and **in that** the material out of which the retroreflective elements (48; 48') are made has a refractive index with a value in a range of about 1.5 to about 2.5 or a refractive index with a value in a range of about 2.5 to about 3.4.

2. Marker element in accordance with Claim 1, **characterized in that** the retroreflective elements (48; 48') are constructed in the shape of spheres (50; 50'),
wherein, in particular, the spheres (50; 50') have a diameter in a range of about 10 µm to about 50 µm, in particular a diameter of about 20 µm.

3. Marker element in accordance with any one of the preceding Claims, **characterized in that**
a) the retroreflective elements (48; 48') are made of glass or a plastics material
and/or
b) the layer (86; 86') of the multitude of retroreflective elements (48; 48') is formed as a single layer.

4. Marker element in accordance with any one of the preceding Claims, **characterized in that** the material out of which the retroreflective elements (48; 48') are made has a refractive index with a value of about 1.93 or a value of about 2.9.

5. Marker element in accordance with any one of the preceding Claims, **characterized in that** the multitude of retroreflective elements (48; 48') is each at least partially provided with a coating (52; 52') reflective to electromagnetic radiation (56).

6. Marker element in accordance with Claim 5, **characterized in that**
a) the coating (52; 52') is formed out of a metal, in particular by vapor deposition,
wherein, in particular, the metal is silver or aluminum,
and/or
b) the reflective coating (52') delimits an ellipsoidal or spherical or substantially spherical cavity (98').

7. Marker element in accordance with any of the Claims 5 or 6, **characterized in that**
a) the layer (86; 86') of retroreflective elements is at least partially embedded into the support (92; 92')
and/or
b) the reflective coating (52') is applied to the layer (86') of retroreflective elements (48') which is at least partially embedded into the support (92').

8. Marker element in accordance with any one of the preceding Claims, **characterized in that** the support material is glass or plastics material, wherein, in particular, the plastics material is or contains polymethylmethacrylate (PMMA).

9. Marker element in accordance with any one of the preceding Claims, **characterized in that** the support material has a support material refractive index which is less than the refractive index of the retroreflective elements (48; 48'),
wherein, in particular, the support material refractive index has a value in a range of about 1.3 to about 1.7, in particular a value of about 1.5.

10. Marker element in accordance with any one of the preceding Claims, **characterized in that**
a) the layer (86; 86') of retroreflective elements (48; 48') is planar or substantially planar or defines a section of an ellipse surface or a sphere surface
and/or
b) the marker element (14; 14') is elliptical or sphere-shaped or substantially sphere-shaped,
wherein, in particular, the reflective coating (52') points in the direction or substantially in the direction toward a midpoint (54') of the sphere-shaped or substantially sphere-shaped marker element (14').

11. Marker element in accordance with any one of the preceding Claims, **characterized in that** the marker element (14') is formed in two or more parts, in particular out of two or more marker element parts (88', 90'), wherein, in particular, the two or more marker element parts (88', 90') are configured in the form of a half-shell or substantially in the shape of a half-shell.

12. Marker element in accordance with any one of the preceding Claims, **characterized in that** the reflective coating (52') delimits an ellipsoidal or spherical or substantially spherical cavity (98') and **in that** the cavity (98') is filled with a filling material,
wherein, in particular, the filling material is a plastics material, further in particular a sterilizable plastics material.

13. Referencing unit (12), in particular medical or surgical referencing unit (12), whose position and/or orientation in the room is detectable with a surgical navigation system (10), having at least one surgical marker element (14; 14'), **characterized in that** the at least one marker element (14; 14') is configured in the form of a marker element (14; 14') in accordance with any one of the preceding Claims.

14. Referencing unit (12) in accordance with Claim 13, **characterized by** a support (18) on which the at least one marker element (14; 14') is arranged or formed.

15. Navigation system (10), in particular a medical or surgical navigation system (10), having at least one referencing unit (12) comprising at least three marker elements (14; 14') and having at least one detection device (16) for detecting the position and/or the orientation of the referencing unit (12) in the room, wherein the referencing unit (12) comprises at least one surgical marker element (14; 14'), **characterized in that** at least one marker element (14; 14') is a marker element (14; 14') in accordance with any one of Claims 1 to 12 and/or **in that** the referencing unit (12) is designed in the form of a referencing unit (12) in accordance with either Claim 13 or 14.

## Revendications

1. Elément de marquage (14; 14'), en particulier élément de marquage (14; 14') médical ou chirurgical, pour une unité de référencement (12) d'un système de navigation (10), lequel élément de marquage (14; 14') est réalisé pour réfléchir un rayonnement électromagnétique (56), où l'élément de marquage comprend une couche (86; 86') comprenant une pluralité d'éléments rétroréfléchissants (48; 48') et un support (92; 92') pour la couche (86; 86') d'éléments rétroréfléchissants (48; 48'), **caractérisé en ce que** le support (92; 92') est formé à partir d'un matériau de support transparent pour le rayonnement électromagnétique (56), **en ce qu'**une surface extérieure de l'élément de marquage (14') est formée par le support (92') et **en ce que** le matériau à partir duquel les éléments rétroréfléchissants (48; 48') sont formés présente un indice de réfraction avec une valeur dans un domaine d'environ 1,5 à environ 2,5 ou un indice de réfraction avec une valeur dans un domaine d'environ 2,5 à environ 3,4.

2. Elément de marquage selon la revendication 1, **caractérisé en ce que** les éléments rétroréfléchissants (48; 48') sont réalisés sous forme de billes (50; 50'),
où en particulier les billes (50; 50') présentent un diamètre dans un domaine d'environ 10 µm à environ 50 µm, en particulier un diamètre d'environ 20 µm.

3. Elément de marquage selon l'une des revendications précédentes, **caractérisé en ce que**
a) les éléments rétroréfléchissants (48; 48') sont produits en verre ou en une matière plastique
et/ou
b) la couche (86; 86') est formée en une seule épaisseur à partir de la pluralité d'éléments rétroréfléchissants (48; 48').

4. Elément de marquage selon l'une des revendications précédentes, **caractérisé en ce que** le matériau à partir duquel sont formés les éléments rétroréfléchissants (48; 48') présente un indice de réfraction avec une valeur d'environ 1,93 ou avec une valeur d'environ 2,9.

5. Elément de marquage selon l'une des revendications précédentes, **caractérisé en ce que** la pluralité d'éléments rétroréfléchissants (48; 48') sont munis chacun au moins partiellement d'un revêtement (52; 52') réfléchissant le rayonnement électromagnétique (56).

6. Elément de marquage selon la revendication 5, **caractérisé en ce que**
a) le revêtement (52; 52') est formé à partir d'un métal, en particulier par dépôt en phase vapeur,
où en particulier le métal est l'argent ou l'aluminium,
et/ou
b) le revêtement réfléchissant (52') délimite une cavité ellipsoïdale ou sphérique ou sensiblement sphérique (98').

7. Elément de marquage selon l'une des revendications 5 ou 6, **caractérisé en ce que**
a) la couche (86; 86') d'éléments rétroréfléchissants est noyée au moins partiellement dans le support (92; 92')
et/ou
b) le revêtement réfléchissant (52') est appliqué sur la couche (86') d'éléments rétroréfléchissants (48') au moins partiellement noyée dans le support (92').

8. Elément de marquage selon l'une des revendications précédentes, **caractérisé en ce que** le matériau de support est du verre ou une matière plastique, où en particulier la matière plastique est ou contient du polyméthacrylate de méthyle (PMMA).

9. Elément de marquage selon l'une des revendications précédentes, **caractérisé en ce que** le matériau de support présente un indice de réfraction de matériau de support qui est inférieur à l'indice de réfraction des éléments rétroréfléchissants (48; 48'), où en particulier l'indice de réfraction de matériau de support présente une valeur dans un domaine d'environ 1,3 à environ 1,7, en particulier une valeur d'environ 1,5.

10. Elément de marquage selon l'une des revendications précédentes, **caractérisé en ce que**
a) la couche (86; 86') d'éléments rétroréfléchissants (48; 48') est plate ou sensiblement plate ou définit une section d'une surface elliptique ou d'une surface sphérique
et/ou
b) l'élément de marquage (14; 14') est réalisé sous forme elliptique ou sphérique ou sensiblement sphérique,
où en particulier le revêtement réfléchissant (52') est tourné en direction ou sensiblement en direction d'un centre (54') de l'élément de marquage sphérique ou sensiblement sphérique (14').

11. Elément de marquage selon l'une des revendications précédentes, **caractérisé en ce que** l'élément de marquage (14') est réalisé en deux ou plusieurs parties, en particulier en deux ou plusieurs parties d'élément de marquage (88', 90'),
où en particulier les deux ou plusieurs parties d'élément de marquage (88', 90') sont réalisées sous forme de demi-coquille ou sensiblement sous forme de demi-coquille.

12. Elément de marquage selon l'une des revendications précédentes, **caractérisé en ce que** le revêtement réfléchissant (52') délimite une cavité (98') ellipsoïdale ou sphérique ou sensiblement sphérique et **en ce que** la cavité (98') est remplie d'un matériau de remplissage, où en particulier le matériau de remplissage est une matière plastique, plus particulièrement une matière plastique stérilisable.

13. Unité de référencement (12), en particulier unité de référencement médicale ou chirurgicale (12), dont la position et/ou l'orientation dans l'espace peut être détectée avec un système de navigation chirurgical (10), avec au moins un élément de marquage chirurgical (14; 14'), **caractérisée en ce que** le au moins un élément de marquage (14; 14') est réalisé sous la forme d'un élément de marquage (14; 14') selon l'une des revendications précédentes.

14. Unité de référencement (12) selon la revendication 13, **caractérisée par** un support (18) sur lequel est disposé ou réalisé le au moins un élément de marquage (14; 14').

15. Système de navigation (10), en particulier système de navigation médical ou chirurgical (10), avec au moins une unité de référencement (12) comprenant au moins trois éléments de marquage (14; 14') et avec au moins un dispositif de mise en évidence (16) pour détecter la position et/ou l'orientation de l'unité de référencement (12) dans l'espace, où l'unité de référencement (12) comprend au moins un élément de marquage chirurgical (14; 14'), **caractérisé en ce qu'**au moins un élément de marquage (14; 14') est un élément de marquage (14; 14') selon l'une des revendications 1 à 12 et/ou **en ce que** l'unité de référencement (12) est réalisée sous la forme d'une unité de référencement (12) selon la revendication 13 ou 14.
